# EUROPEAN PATENT APPLICATION

(11) **EP 1 649 815 A1**
(43) Date of publication of application: **26.04.2006**
(21) Application number: 04748226.0
(22) Date of filing: 28.07.2004
(51) Int. Cl.: A61B 17/02

(54) **ULTRASONIC THERAPEUTIC INSTRUMENT AND THERAPEUTIC METHOD UTILIZING THE SAME**

(30) Priority: 28.07.2003 JP 2003281064
(71) Applicant: Olympus Corporation, Tokyo 151-0072 (JP)
(72) Inventor: ISHIKAWA, Manabu c/o OLYMPUS Serv. Co. Ltd., Hachioji-shi, Tokyo 192-8512 (JP)
(74) Representative: von Hellfeld, Axel
(86) International application number: PCT/JP2004/011121
(87) International publication number: WO 2005/009249

(57) **Abstract**

An ultrasonic treatment device includes a handpiece (2), a probe (8), a sheath (10), and a lifting operation portion (S). A distal end portion of the probe (8) has a puncture portion (a) that transmits ultrasonic oscillation from an ultrasonic oscillator (3) to a living-body wall part (H) and punctures the living-body wall part (H). The sheath (10) includes a cylindrical member having a distal end portion and a proximal end portion. The cylindrical member is detachably fitted on the probe (8) such that only the distal end portion of the probe (8) is exposed and the other part of the probe (8) is covered. The sheath (10) together with the probe (8) is passed through the living-body wall part (H) by puncture. The lifting operation portion (S) is provided on the sheath (10). The lifting operation portion (S) is engaged with the living-body wall part (H) at a time of performing a pull-up operation of pulling up the sheath (10) in a direction opposite to a direction of puncture through the living-body wall part (H), thereby performing an operation of lifting the living-body wall part (H).

## Description

### Technical Field

The present invention relates to an ultrasonic treatment device that is inserted in the body and used in a treatment for, e.g. lifting the body wall or a living-body wall part of, e.g. a tubular organ, and to a treatment method using the ultrasonic treatment device.

### Background Art

There are known an ultrasonic coagulation/cutting device, an ultrasonic suction device, etc., which include ultrasonic oscillators that are ultrasonic generating elements. These devices have widely been used in the fields of surgical operations, cerebral surgery operations and ophthalmological operations. The ultrasonic oscillator, in general terms, comprises an ultrasonic generating element, a horn, and a casing.

In the ultrasonic oscillator, a plurality of ultrasonic generating elements are stacked. The ultrasonic generating element is, in typical examples, formed of barium zirconate titanate. In general, the number of stacked ultrasonic generating elements is freely chosen in accordance with the output of the ultrasonic oscillator. The number of ultrasonic generating elements, which are stacked in the ultrasonic oscillator, is two to four, for instance.

A horn, which is a member that is so shaped as to amplify ultrasonic oscillation, is provided at a distal end portion of the stacked structure of the ultrasonic generating elements. The horn is formed of, e.g. titanium 6A14 vanadium. The ultrasonic generating elements and the horn are integrally coupled by a back-mass that is used at the rear end portion of the ultrasonic generating elements. A distal end portion of the horn is processed to have such a shape as to enable screw coupling.

A cable for supplying power to the ultrasonic generating element is integrally, or detachably, provided at the rear end of the ultrasonic oscillator. A cable extending from the ultrasonic oscillator is connected to the ultrasonic oscillation device, and an output signal from the ultrasonic oscillation device is input to the ultrasonic generating elements.

An ultrasonic transmission member of a given kind, which is called a probe, is coupled by screwing to the distal end of the ultrasonic oscillator (the distal end of the horn). The ultrasonic transmission member is processed in such a shape as to further increase the amplitude at the distal end of the horn. The ultrasonic transmission member is designed to output a highest ultrasonic oscillation at the distal end of the ultrasonic transmission member.

For example, an ultrasonic coagulation/cutting device, which makes use of the above-described ultrasonic oscillation technique, is used as medical equipment for coagulating a living tissue at low temperatures and performing cutting while preventing bleeding.

Jpn. Pat. Appln. KOKOKU Publication No. 5-57863 and Jpn. Pat. Appln. KOKAI Publication No. 2002-177293 disclose ultrasonic trocar systems. These systems are characterized in that ultrasonic oscillation is used to prevent bleeding, thereby facilitating puncture of a body wall.

Partial resection of the stomach, for instance, is one of endoscopic surgical techniques, which are performed while observing the inside of the body by an endoscope. In this technique, to begin with, a trocar is inserted through the body wall. Through the trocar, a treatment instrument of a given kind is inserted into the body cavity. Further, a diseased part of the stomach is punctured by an instrument with a pointed distal end, or by a needle-like instrument, and the diseased part of the stomach is lifted. Then, the diseased part is cut and removed using a cutting instrument that is called "automatic suturing device." There are some problems with this technique. That is, in order to puncture the stomach with a treatment instrument, it is necessary to make a trocar pierce the abdominal wall and to position the trocar there. Even in the case where the trocar is hardly used for other treatment, it is necessary to perform a special procedure for preparing the trocar, performing puncture with the trocar, and positioning the trocar. This imposes a load on the operator, and careful handling is required for the puncture.

A tubular organ, such as the stomach, has a firm structure of living tissues, and is always moving. It is very difficult, therefore, to puncture the organ with the trocar. Moreover, the procedure for smoothly passing the trocar into the organ requires a very high skill level. If unintended bleeding occurs during the procedure for puncturing the organ with the trocar, the point of bleeding, in many cases, is present within the lumen. Consequently, hemostasis is a very difficult, time-consuming treatment.

There are similar problems when partial resection of the stomach is performed in endoscopic surgical operations. In the partial resection of the stomach, a procedure for lifting the diseased part of the stomach and keeping the lifted state is performed. Thereby, it becomes possible to increase the range of visual field of the endoscope and to facilitate the resection/removal of the diseased part while observing it.

However, the treatment instrument for puncture the stomach differs from the treatment instrument for lifting the diseased part. Consequently, in the case where the procedure for puncturing the stomach and the procedure for lifting the diseased part are performed, it is necessary to change the treatment instrument each time these treatments are performed, or to prepare a trocar for puncture and a trocar for lifting the diseased part. In either case, the load on the operator increases.

The present invention has been made in consideration of the above circumstances, and the object of the invention is to provide an ultrasonic treatment device, which can perform smooth puncture in the body wall and tubular organ while preventing bleeding, can surely lift the body wall or a living-body wall part of a tubular organ, can perform treatment more safely, and can greatly decrease the time for operations, and to provide a treatment method using the ultrasonic treatment device.

### Disclosure of Invention

According to the present invention, there is provided an ultrasonic treatment device characterized by comprising:
a handpiece accommodating an ultrasonic oscillator that generates ultrasonic oscillation;
a probe including a shaft-shaped oscillation transmission member having a distal end portion and a proximal end portion, the proximal end portion being connected to the ultrasonic oscillator, the distal end portion having a puncture portion that transmits the ultrasonic oscillation from the ultrasonic oscillator to a living-body wall part and punctures the living-body wall part;
a sheath including a cylindrical member having a distal end portion and a proximal end portion, the cylindrical member being detachably fitted on the probe such that only the distal end portion of the probe is exposed and the other part of the probe is covered, the sheath together with the probe being passed through the living-body wall part by puncture; and
a lifting operation portion provided on the sheath, the lifting operation portion being engaged with the living-body wall part at a time of performing a pull-up operation of pulling up the sheath in a direction opposite to a direction of puncture through the living-body wall part, thereby performing an operation of lifting the living-body wall part.

The ultrasonic treatment device according to the first aspect, characterized in that the probe has at least a length that is needed for penetration through the living-body wall part including a body wall and an internal organ.

The ultrasonic treatment device according to the first aspect, characterized in that the lifting operation portion includes an anti-slip portion with a continuous projection-and-recess shape, which is provided on an outer peripheral surface of the sheath.

The ultrasonic treatment device according to the first aspect, characterized in that the lifting operation portion includes:
a bend section that is provided on an outer peripheral surface of the sheath, the bend section including a plurality of bend pieces that are juxtaposed in an axial direction of the sheath, the bend pieces being rotatably coupled to each other;
an operation wire having a distal end portion and a proximal end portion, the distal end portion of the wire being connected to that one of the bend pieces, which is disposed at a foremost position in the axial direction of the sheath, the proximal end portion of the wire extending toward the proximal end portion of the sheath; and
a bend operation part that is provided on the proximal end portion side of the sheath, the bend operation part executing an operation of pulling the wire, thereby bending the bend section.

The ultrasonic treatment device according to the first aspect, characterized in that the lifting operation section includes a deformable portion in which a plurality of slits that extend in an axial direction of the sheath are juxtaposed in a circumferential direction of the sheath, the deformable portion changing a state of wall portions, which are provided between the slits, between a non-deformed state in which the wall portions are in a straight shape and a projecting state in which the wall portions project outward, thereby varying a maximum outside diameter of the sheath.

According to the present invention, there is provided an ultrasonic treatment method characterized by comprising:
an insertion step of inserting a probe, which is capable of transmitting ultrasonic oscillation to a living-body wall part, and a sheath that covers the probe, through the living-body wall part; and
a lifting step of lifting the living-body wall part by a lifting operation portion for lifting the living-body wall part, the lifting operation portion being provided on the sheath, which is inserted into the body through the living-body wall part in the insertion step.

The ultrasonic treatment method according to the sixth aspect, characterized in that the insertion step includes:
a first insertion step of inserting the probe, which is capable of transmitting ultrasonic oscillation to the living-body wall part, and the sheath that covers the probe, through a body wall part of the living-body wall part;
a second insertion step of inserting the probe and the sheath, which are inserted into the body through the body wall part in the first insertion step, into an internal organ that is located inside the body wall part; and
a lifting step of lifting the internal organ by a lifting operation portion for lifting the internal organ, the lifting operation portion being provided on the sheath, which is inserted in the second insertion step.

In the present invention, the body wall and a living-body wall part of, e.g. a tubular organ are smoothly punctured with a probe that makes use of ultrasonic oscillation while preventing bleeding. Further, the body wall and a living-body wall part of, e.g. a tubular organ are surely lifted and held by the lifting operation portion of the sheath. Thereby, the safety of subsequently treatment is enhanced, and the time for operations is greatly reduced.

### Brief Description of Drawings

FIG. 1A schematically shows the whole structure of an ultrasonic treatment device according to a first embodiment of the present invention;
FIG. 1B is a side view showing the structure of an anti-slip portion of the ultrasonic treatment device according to the first embodiment;
FIG. 2 is a view for illustrating a state in which the ultrasonic treatment device according to the first embodiment is actually inserted in the body of a patient;
FIG. 3A is a view for illustrating a state in which the ultrasonic treatment device according to the first embodiment is to be inserted in the body of the patient;
FIG. 3B is a view for illustrating a state in which the ultrasonic treatment device according to the first embodiment is passed through the body wall of the patient;
FIG. 3C is a view for illustrating a state in which the ultrasonic treatment device according to the first embodiment is passed through an organ within the body of the patient;
FIG. 4A is a view for illustrating a state in which a probe is removed from a sheath of the ultrasonic treatment device according to the first embodiment;
FIG. 4B is a view for illustrating a state in which a diseased part is treated while a part of the organ is being lifted, at the time of using the ultrasonic treatment device according to the first embodiment;
FIG. 5 is a view that shows the state of use of an ultrasonic treatment device according to a second embodiment of the present invention;
FIG. 6 is a view that shows the state of use of an ultrasonic treatment device according to a third embodiment of the present invention;
FIG. 7 shows a modification of the ultrasonic treatment device according to the third embodiment;
FIG. 8 is a view that shows the state of use of an ultrasonic treatment device according to a fourth embodiment of the present invention; and
FIG. 9 shows a modification of the ultrasonic treatment device according to the fourth embodiment.

Best Mode for Carrying Out the Invention

### [First Embodiment]

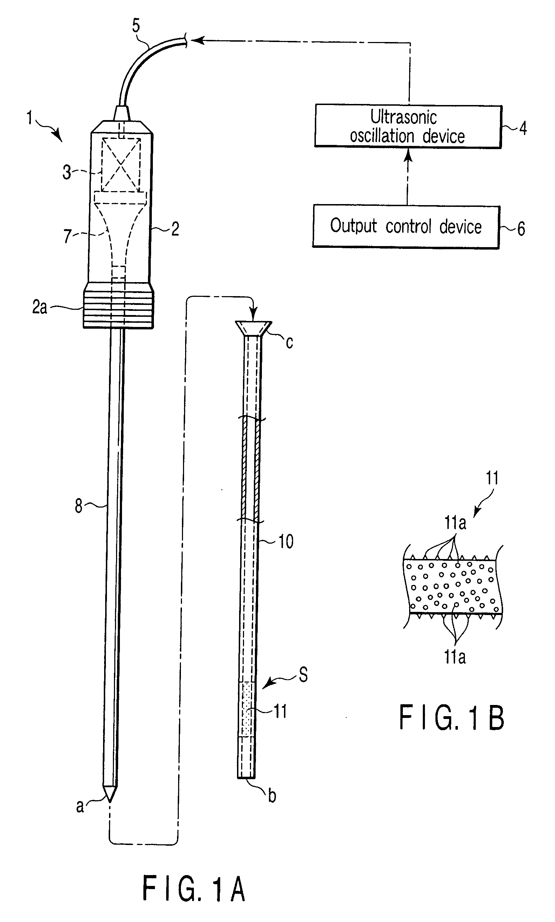

FIG. 1A through FIG. 4B show a first embodiment of the present invention. FIG. 1A shows the entire structure of an ultrasonic treatment device 1. The ultrasonic treatment device 1 includes a handpiece 2 that is held by the hand of an operator. The handpiece 2 includes an ultrasonic oscillator 3 that generates ultrasonic oscillation. The ultrasonic oscillator 3 is connected to an ultrasonic oscillation device 4 via a cable 5. Energy is supplied from the ultrasonic oscillation device 4 to the ultrasonic oscillator 3 via the cable 5.

An output control device 6, such as a foot switch or a hand switch, is connected to the ultrasonic oscillation device 4. The output of the ultrasonic device 4 is controlled by the output control device 6.

The ultrasonic oscillator 3 comprises a plurality of ultrasonic generating elements, which are formed of, e.g. barium zirconate titanate. A horn 7, which is formed of, e.g. titanium 6A14 vanadium, is integrally coupled to the ultrasonic oscillator 3. Further, a probe 8, which is an ultrasonic oscillation transmission member, is connected by screwing to a distal end portion of the horn 7. The probe 8 has at least a length that is needed for penetration through the body wall or organ of the patient. A tapering, sharp puncture portion a is formed at a distal end portion of the probe 8.

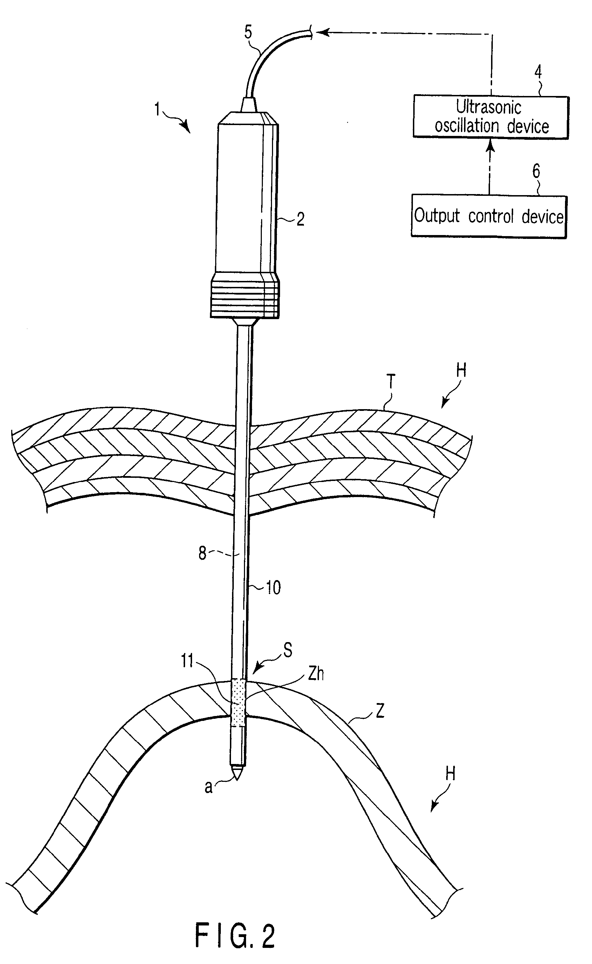

The probe 8 is detachably inserted in a sheath 10. The sheath 10 is cylindrical and has an inside diameter that is substantially equal to the outside diameter of the probe 8. The length of the sheath 10 is set as follows. That is, when the probe 8 is inserted in the sheath 10, as shown in FIG. 2, only the puncture portion a at the distal end is exposed on the front side of the sheath 10, and the other part of the probe 8 is covered in the sheath 10. In this manner, the length of the sheath 10 is set.

It is preferable to select, as the material of the sheath 10, a bio-compatible material with high heat resistance and good slippage relative to the probe 8, such as PTFE (polytetrafluoroethylene).

A lifting operation portion S is provided at a portion near the distal end portion of the sheath 10. In this embodiment, the lifting operation portion S has an anti-slip portion 11. The anti-slip portion 11 is provided over a predetermined length on a peripheral surface portion of the sheath 10, which is located at a predetermined distance from a distal-end edge b of the sheath 10. The anti-slip portion 11 includes a great number of minute projections 11a. Thus, the outer peripheral surface of the sheath 10 has a projection-and-recess shape at the region of the anti-slip portion 11.

As will be described later, the sheath 10 is passed through the body wall T of the patient, or a living-body wall part H of an organ Z, for instance. Thereby, the many minute projections 11a of the anti-slip portion 11 are engaged with the body wall T or organ Z, thereby preventing a slip. As a result, if the sheath 10 is raised in the state in which the anti-slip portion 11 is engaged with the body wall T or organ Z, the body wall T or organ Z can be lifted.

As regards such minute projections 11a of the anti-slip portion 11, no frictional heat occurs between the probe 8 and sheath 10 in the state in which ultrasonic oscillation is caused by the probe 8, and no thermal adverse effect occurs. In addition, when the sheath 10 passes through the body wall T or organ Z, the anti-slip portion 11 has no high resistance.

A proximal end portion of the sheath 10 is provided with an insertion guide portion c with a diameter gradually increasing in a funnel shape. The insertion guide portion c serves as a guide when the sheath 10 is to be fitted on the probe 8, or as a guide when a suction tube is to be inserted in the sheath 10, as will be described later.

An O-ring attachment groove (not shown) is formed in an outer peripheral surface of the insertion guide portion c of the sheath 10. An O-ring (not shown) is fitted in the O-ring attachment groove. When the sheath 10 is fitted on the probe 8 and the insertion guide portion c is inserted in the cylindrical connection portion 2a at the lower end of the handpiece 2, the O-ring of the insertion guide portion c is put in pressure contact with the inner peripheral surface of the cylindrical connection portion 2a. Thereby, the insertion guide portion c of the sheath 10 is engaged with the handpiece 2, and a gap between the sheath 10 and the cylindrical connection portion 2a of the handpiece is sealed.

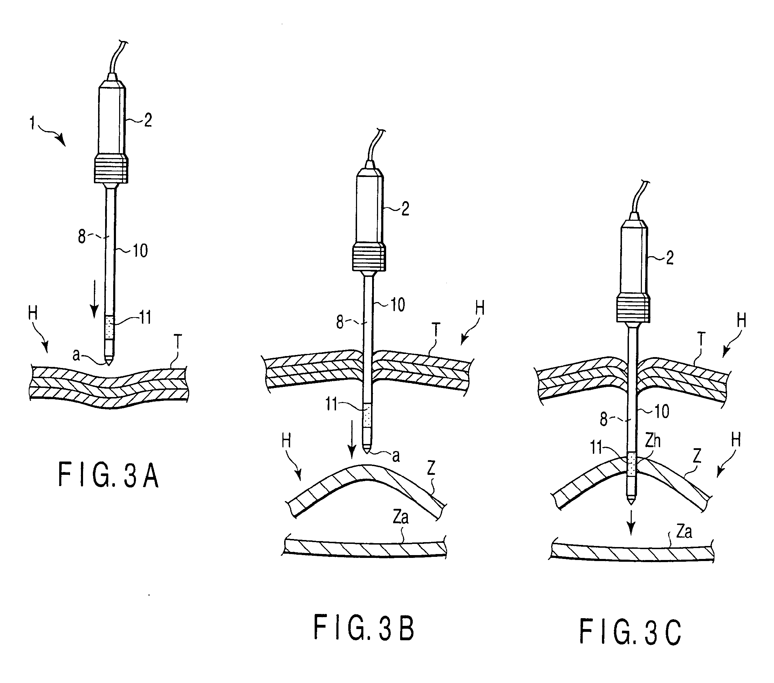

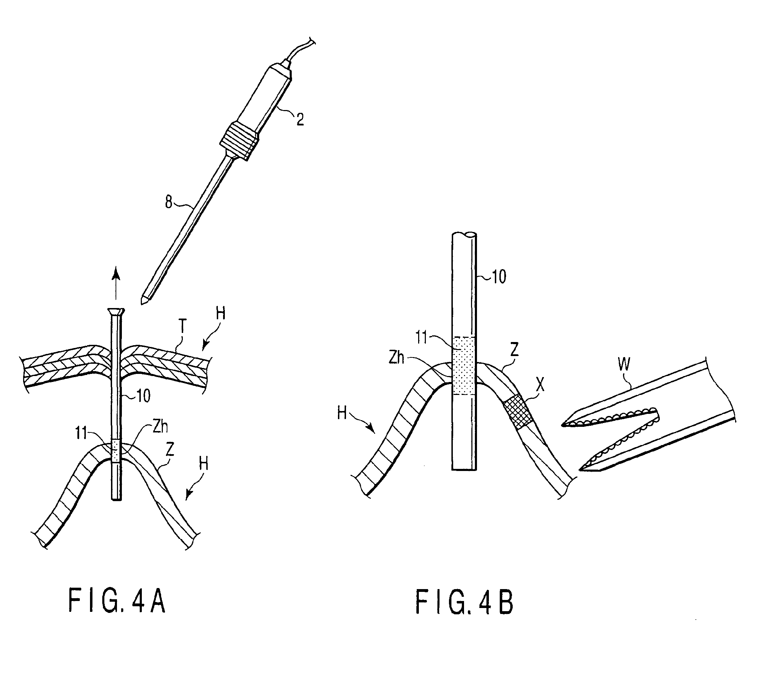

Next, the operation of the ultrasonic treatment device 1 is described. FIG. 2 is a cross-sectional view for illustrating a state in which the ultrasonic treatment device 1 is actually inserted in the body of a patient. FIG. 3A, FIG. 3B, FIG. 3C, FIG. 4A and FIG. 4B illustrate, in succession, actual operational steps at a time of ultrasonic treatment.

When the ultrasonic treatment device 1 is used, the probe 8 is inserted and fitted in advance in the sheath 10 (see FIG. 2).

Then, for example, by operating the output control device 6 that is a foot switch, the output of the ultrasonic oscillation device 4 is controlled. Driving energy that is generated by the ultrasonic oscillation device 4 is input to the ultrasonic oscillator 3 within the handpiece 2 via the cable 5. At this time, in the ultrasonic oscillator 3, ultrasonic oscillation corresponding to the input is generated. The generated ultrasonic oscillation is amplified by the horn 7.

The oscillation is further amplified in the probe 8 at the distal end of the horn 7, and the oscillation takes a maximum value at the distal end of the probe 8. At this time, only the distal-end puncture portion a of the probe 8 is exposed from the sheath 10, and the other major part of the probe 8 is covered in the sheath 10. Thereby, when the procedure for inserting the probe 8 into the body of the patient is performed, the range of area, at which the probe 8 contacts the living-body wall part H, is limited to the distal-end puncture portion a. As a result, it is possible to prevent ultrasonic oscillation energy from being excessively applied to an unintended area of the living-body wall part H.

Next, the actual ultrasonic treatment using the ultrasonic treatment device 1 is described in the order of operational steps.

To start with, the operator holds the handpiece 2 and guides the distal-end puncture portion a of the probe 8, on which the sheath 10 is fitted, to a part to be treated. At this time, as shown in FIG. 3A, the distal-end puncture portion a of the probe 8 is approached to the body wall T in the vicinity of a diseased part to be treated.

As shown by an arrow in FIG. 3A, the puncture portion a of the probe 8 is pushed toward the body wall T. Thereby, as shown in FIG. 3B, the puncture portion a of the probe 8 is passed through the body wall T. In this case, since ultrasonic oscillation energy is applied to the puncture portion a of the probe 8, no bleeding occurs at the time of puncture, the force needed for puncture is very weak, and smooth puncture can be performed. Hence, since the degree of tenting of the body wall T (the state of lowering of the wall) is small, the distance between the body wall T and organ Z does not excessively decrease and safer puncture can be performed.

When the body wall T is punctured with the puncture portion a of the probe 8, the sheath 10, together with the probe 8, is inserted through the body wall T, as shown in FIG. 3B. Thereby, a first insertion step of passing the sheath 10 together with the probe 8 through the body wall T is performed.

The distal-end puncture portion a of the probe 8, which has been passed through the body wall T in the first insertion step, is pushed toward the organ Z, that is, the diseased part, as indicated by an arrow in FIG. 3B.

Subsequently, as shown in FIG. 3C, a second insertion step is executed to insert the distal-end puncture portion a of the probe 8 through a part near the diseased part of the organ Z to be treated, for instance, the stomach. In the second insertion step, too, the sheath 10 together with the probe 8 is passed through the wall of the organ Z. Thereby, like the body wall T, the organ Z is prevented from bleeding by ultrasonic oscillation, and smooth puncture can be performed.

The length of the probe 8 and sheath 10 is designed to be sufficiently greater than the thickness of the body wall T and the organ Z. The probe 8 and sheath 10 have at least a length that is needed for penetration through the body wall T and organ Z. It is thus possible to easily perform puncture into the inside of the organ Z.

Further, the puncture procedure in the second insertion step is performed until the anti-slip portion 11 of the sheath 10 penetrates the wall part of the organ Z. At this time, as mentioned above, the many minute projections 11a of the anti-slip portion 11 do not cause resistance when the sheath 10 penetrates the body wall T and organ Z.

During the puncture procedure in the second insertion step, if it is confirmed that the anti-slip portion 11 that is provided near the distal end portion of the sheath 10 has reached the inside of the organ Z, the supply of driving energy to the ultrasonic oscillator 3 is stopped. Thus, the puncture procedure in the second insertion step by means of the probe 8 using ultrasonic oscillation is completed.

When the organ Z is punctured with the probe 8 and sheath 10, it may be possible to perform in advance a treatment of holding and lifting the organ Z using a treatment instrument of a desired kind. In this case, the possibility that the distal end of the probe 8 contacts a wall part (bottom) Za of the organ Z, which is opposed to the punctured wall part, decreases, and safer puncture can be performed.

When the puncture procedure with the probe 8 in the second insertion step is completed, the distal end of the probe 8 is passed into the inside of the organ Z, and also the distal end of the sheath 10 is passed into the inside of the organ Z. In this state, a procedure for drawing the handpiece 2 out of the sheath 10, as shown in FIG. 4A, is performed.

If the handpiece 2 is removed from the sheath 10, the sheath 10 alone penetrates the body wall T and organ Z, and only the distal end portion of the sheath 10 is positioned within the organ Z.

Then, as indicated by an arrow in FIG. 4A, an operation is performed to pull up the positioned sheath 10 in a direction opposite to the direction of penetration into the body. During the operation of pulling up the sheath 10, the sheath 10 smoothly moves through a puncture hole Zh in the organ Z until the anti-slip portion 11 of the sheath 10 reaches the wall part of organ Z where the puncture hole Zh is made.

When the anti-slip portion 11 passes through the puncture hole Zh in the wall part of the organ Z, as shown in FIG. 4A, the many minute projections 11a of the anti-slip portion 11 stick in the inner peripheral surface of the puncture hole Zh in the wall part of the organ Z. In other words, the many minute projections 11a of the anti-slip portion 11 are caught in the inner peripheral surface of the puncture hole Zh in the organ Z, thus preventing a slip. Thereby, a resistance is caused when the anti-slip portion 11 is moved in a direction in which the anti-slip portion 11 is drawn out of the puncture hole Zh in the wall part of the organ Z.

In this state, the operation for further pulling up the sheath 10 is continued. At this time, the sheath 10 moves in a direction opposite to the direction of puncture in the state in which the many projections 11a of the anti-slip portion 11 are caught in the inner peripheral surface of the puncture hole Zh of the organ Z. Accordingly, the peripheral wall part of the puncture hole Zh of the organ Z is pulled up together with the sheath 10. As a result, the peripheral wall part of the puncture hole Zh of the organ Z is lifted toward the body wall T.

As has been described above, in the operation for partial resection of the stomach, a diseased part X of the stomach is lifted by the sheath 10, as shown in FIG. 4B, and this state is kept. Thereby, the range of visual field of the endoscope is widened, and the diseased part, while being confirmed, is treated. In this case, for example, the diseased part X of the stomach can be cut and removed by using a treatment device W such as an automatic suturing device. Thus, the treatment necessary for the diseased part can smoothly be performed.

In addition, when a part of the organ Z is lifted, the anti-slip portion 11 of the sheath 10 engages the organ Z and this contributes to prevention of bleeding.

Besides, the safety is further enhanced if the endoscope is placed in advance within the stomach when the operation for partial resection of the stomach is to be performed, and the probe 8 and sheath 10 are inserted through the stomach while the inside of the stomach is being observed.

A still safer treatment can be performed if a small hole (not shown) is made in the handpiece 2 and probe 8, a fine scope is passed through the small hole, and the organ Z is punctured with the probe 8 while observation through the scope is being performed.

After all treatments including the procedure for partial resection of the stomach are completed, an operation is performed to pull up the sheath 10 in a direction opposite to the direction of puncture with a still greater operation force than that for lifting the organ Z. At this time, the anti-slip portion 11 is forcibly drawn out of the peripheral wall part of the puncture hole Zh in the organ Z. Subsequently, the anti-slip portion 11 of the sheath 10 is easily drawn out of the inside of the patient's body through the body wall T. Thus, the surgical operation is completed.

The embodiment with the above-described structure has the following advantageous effects. In the ultrasonic treatment device of the present embodiment, the procedure for passing the probe 8 through the body wall T and organ Z successively is the puncture procedure using ultrasonic oscillation energy. Thus, the body wall T and organ Z can smoothly be punctured with the probe 8 using ultrasonic oscillation while bleeding is being prevented.

Further, when the puncture procedure with the probe 8 is performed, only the distal-end puncture portion a of the probe 8 is exposed and the other part of the probe 8 is covered with the sheath 10. Thus, ultrasonic oscillation energy is not excessively applied to the living-body wall part.

Furthermore, the anti-slip portion 11 is provided on the distal-end side of the sheath 10. The sheath 10, which is passed through, and positioned in, the organ Z, is pulled up in a direction opposite to the direction of puncture. Thereby, the wall part of the tubular organ Z can surely be lifted by the anti-slip portion 11. Thus, as shown in FIG. 4B, the diseased part X of the stomach is lifted by the sheath 10 and this state is kept. Thereby, the range of visual field of the endoscope is widened, and the diseased part, while being confirmed, is treated. It is possible, therefore, to more safely and smoothly perform, for example, the treatment for cutting and removing the diseased part X of the stomach by using the treatment device W such as an automatic suturing device. The time for the surgical operation can remarkably be reduced.

### [Second Embodiment]

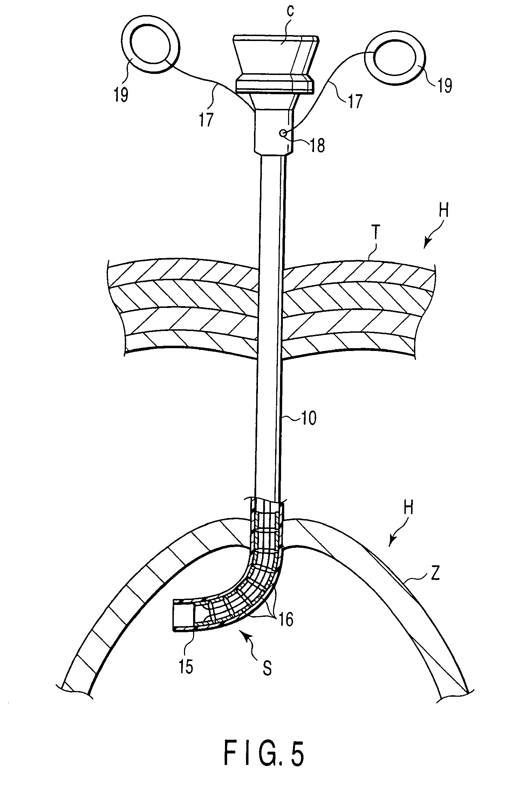

FIG. 5 shows a second embodiment of the ultrasonic treatment device 1 of the present invention. The basic structure of the ultrasonic treatment device 1 of this embodiment is the same as that of the first embodiment (see FIG. 1A to FIG. 4B) except for the sheath 10. FIG. 5 shows only the sheath 10. A description of the other structural parts, which are common to those in FIG. 1, is omitted here.

In this embodiment, a lifting operation portion S of the sheath 10 includes a bend section 15 that is disposed at the distal end of the sheath 10. The bend portion 15 includes a plurality of bend pieces 16 that are juxtaposed along the axis of the sheath 10. The respective bend pieces 16 are rotatably coupled to one another by means of rotational support members such as rivets.

Distal end portions of two operation wires 17 are connected to the foremost one of the bend pieces 16 of the bend section 15. The two operation wires 17 are positioned at portions of the bend piece 16, which are circumferentially distanced by about 180° .

A proximal end portion of each wire 17 is extended to the insertion guide portion c at the proximal end of the sheath 10. Further, two wire insertion holes 18 are made in the proximal end portion of the sheath 10. These two wire insertion holes 18 are positioned at portions of the proximal end portion of the sheath 10, which are circumferentially distanced by about 180° .

The two operation wires 17 are drawn out through the wire insertion holes 18 and connected to operation rings 19. In the state in which both of the two operations wires 17 are not operated and pulled, the standby state is kept. In this state, the bend section 15 is kept in a straight shape.

If one of the two operation rings 19 is pulled, the bend section 15 is bent. At this time, the foremost bend piece 16 of the bend section 15 of the sheath 10 is pulled in the direction of the pulling operation by means of the operation wire 17. Thus, the bend pieces 16 of the bend section 15 are rotated about the associated rotational support members, and the whole bend section 15 of the sheath 10 is bent substantially in a J-shape. The sheath 10 has the above-described structure.

Next, the operation of the ultrasonic treatment device 1 according to this embodiment with the above structure is described. Like the first embodiment, when the ultrasonic treatment device 1 of this embodiment is used, the probe 8, on which the sheath 10 is detachably fitted, is passed into the body. Similarly with the first embodiment, only the distal-end puncture portion a of the probe 8 is exposed and the other part of the probe 8 is covered in the sheath 10.

When the ultrasonic treatment device 1 of this embodiment is used, the probe 8 and sheath 10 are inserted through the body wall T and organ Z in the same procedure as in the first embodiment (the above-described first insertion step and second insertion step).

Following the completion of the second insertion step, the procedure for removing the sheath 10 from the probe 8 is performed. Then, one of the rings 19 at the external ends of the operation wires 17 is held by the finger, and the operation wire 17 is pulled.

At this time, the foremost bend piece 16 of the bend section 15 of the sheath 10 is pulled in the direction of the pulling operation by means of the operation wire 17. Thus, the bend pieces 16 of the bend section 15 are rotated about the associated rotational support members, and the whole bend section 15 of the sheath 10 is bent substantially in a J-shape.

In this state, the sheath 10 is pulled up. When the sheath 10 is pulled up, the bend section 15 that is in the bent state is hooked at a part of the organ Z. Thus, by continuing the operation of pulling up the sheath 10, the operation of lifting the part of the organ Z by the bend section 15 is performed.

The embodiment with the above structure has the following advantageous effects. The ultrasonic treatment device 1 of the present embodiment has the same advantageous effects as with the first embodiment. Further, in the present embodiment, the bend section 15, which serves as the lifting operation portion S at the distal-end side of the sheath 10, is provided. Thus, by pulling up the sheath 10, which is passed through and positioned in the organ Z, in a direction opposite to the direction of puncture, the wall part of the tubular organ Z can surely be raised by the bend section 15. Thereby, the range of visual field of the endoscope is widened, and the diseased part, while being confirmed, is treated. It is possible, therefore, to more safely and smoothly perform, for example, the treatment for cutting and removing the diseased part X of the stomach by using the treatment device W such as an automatic suturing device. The time for the surgical operation can remarkably be reduced.

### [Third Embodiment]

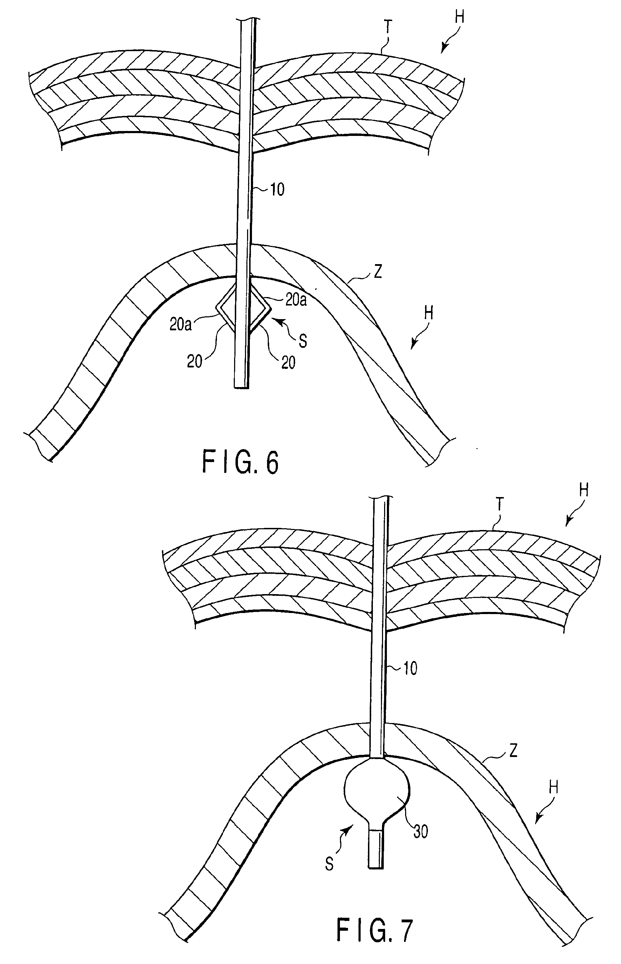

FIG. 6 shows a third embodiment of the ultrasonic treatment device 1 of the present invention. In this embodiment, the lifting operation portion S of the sheath 10 is altered, as will be described below. The basic structure, except for the sheath 10, is the same as that of the first embodiment (see FIG. 1A to FIG. 4B). FIG. 6 shows only the sheath 10. A description of the other structural parts, which are common to those in FIG. 1, is omitted.

In this embodiment, a lifting operation portion S of the sheath 10 is configured to have a plurality of slits (linear cut openings) 20a. The slits 20a extend in the axial direction of the sheath 10 and are juxtaposed in the circumferential direction of the sheath 10 at a position that is away from the distal end of the sheath 10 by a predetermined distance. Wall parts between the slits 20a are provided with deformable portions 20 that are deformable between a non-deformed state in which the deformable portions 20 are in a straight shape, and a projecting state in which the deformable portions 20 resiliently project outward.

Next, the operation of the ultrasonic treatment device 1 according to this embodiment with the above structure is described. Like the first embodiment, when the ultrasonic treatment device 1 of this embodiment is used, the probe 8, on which the sheath 10 is detachably fitted, is passed into the body. Similarly with the first embodiment, only the distal-end puncture portion a of the probe 8 is exposed and the other part of the probe 8 is covered in the sheath 10.

When the ultrasonic treatment device 1 of this embodiment is used, the probe 8 and sheath 10 are inserted through the body wall T and organ Z in the same procedure as in the first embodiment (the above-described first insertion step and second insertion step). At this time, the deformable portions 20 of the sheath 10 are kept in the non-deformed state in which the wall parts between the slits 20a are in the straight shape. Thus, the deformable portions 20 of the sheath 10 can smoothly be inserted through the body wall T and organ Z.

Following the completion of the second insertion step, the procedure for removing the sheath 10 from the probe 8 is performed. Thereby, the sheath 10 alone penetrates the body wall T and organ Z, and only the distal end portion of the sheath 10 is positioned within the organ Z.

Subsequently, an operation is performed to pull up the positioned sheath 10 in a direction opposite to the direction of penetration into the body. During the operation of pulling up the sheath 10, the sheath 10 smoothly moves through the puncture hole Zh in the organ Z until the deformable portions 20 of the sheath 10 reach the wall part of organ Z where the puncture hole Zh is made.

When the deformable portions 20 pass through the puncture hole Zh in the wall part of the organ Z, the deformable portions 20 deform into the projecting state in which the wall parts between the slits 20a resiliently project outward. The distance between the apices of the deformable portions 20 is greater than the diameter of the sheath 10.

In this state, the operation for pulling up the sheath 10 is performed. At the time of pulling up the sheath 10, the deformable portions 20 that are deformed in the projecting state are hooked at a part of the organ Z. Thus, by continuing the operation for pulling up the sheath 10, a lifting step is performed to lift the part of the organ Z by the deformable portions 20.

The embodiment with the above structure has the following advantageous effects. The ultrasonic treatment device 1 of the present embodiment has the same advantageous effects as with the first embodiment. Further, in the present embodiment, the deformable portions 20, which serve as the lifting operation portion S at the distal-end side of the sheath 10, are provided at the wall parts between the slits 20a juxtaposed in the circumferential direction. The deformable portions 20 are deformable between the non-deformed state in which the deformable portions 20 are in the straight shape, and the projecting state in which the deformable portions 20 resiliently project outward. When the sheath 10, which is passed through and positioned in the organ Z, is pulled up in a direction opposite to the direction of puncture into the body, the wall part of the tubular organ Z can easily be lifted by the deformable portions 20 of the sheath 10. Thereby, the range of visual field of the endoscope is widened, and the diseased part, while being confirmed, is treated. It is possible, therefore, to more safely and smoothly perform the treatment for cutting and removing the diseased part X of the stomach by using the treatment device W such as an automatic suturing device. The time for the surgical operation can remarkably be reduced.

In the present embodiment, the deformable portions 20 of the sheath 10 are normally kept in the non-deformed state in which the wall parts between the slits 20a are in the straight shape. In addition, when the sheath 10 is pulled up in the direction opposite to the direction of puncture into the body, the deformable portions 20 of the sheath 10 are deformed in the projecting state in which the deformable portions 20 resiliently project outward. Alternatively, the following structural modification may be made. That is, the deformable portions 20 of the sheath 10 may normally be urged in the projecting state in which the deformable portions 20 resiliently project outward. When the probe 8 is passed through the body wall T and organ Z, the deformable portions 20 resiliently extend to have an outside dimension equal to the outside diameter of the sheath 10. Thus, the sheath 10 can smoothly be passed through the body wall T and organ Z.

In this case, if the deformable portions 20 pass through the body wall T and organ Z, the resilient restoring force acts and the deformable portions 20 naturally project as shown in FIG. 6. As a result, if the sheath 10 is pulled up in the pull-up step, the deformable portions 20 are hooked on the organ Z and the part of the organ Z can easily be lifted.

If the sheath 10 is to be drawn out of the body cavity, the sheath 10 is further pulled up. Thereby, the sheath 10 can smoothly be drawn out.

FIG. 7 shows a modification of the sheath 10 according to the third embodiment. In the sheath 10 according to this modification, a balloon 30 is provided as the lifting operation portion S. The balloon 30 is disposed at a position that is away from the distal end portion of the sheath 10 by a predetermined distance. The basic structure of this modification is the same as that of the first embodiment, except for the sheath 10.

The balloon 30 is formed of a material, such as silicone, which is bio-compatible and is easy to expand/contract. The balloon 30 can be expanded when necessary, and can be contracted in other cases. The balloon 30 that is the lifting operation portion S, which is to be described below, functions also as a deformable portion that varies the maximum outside diameter of the sheath 10.

Although not shown, a syringe is attached to a mouthpiece-like inlet that is provided at an upper part of the sheath 10, which is located outside the body cavity. Air is injected in the balloon 30 by a piston operation of the syringe. Thereby, the balloon 30 can be expanded. In addition, by the piston operation of the syringe, the air in the balloon 30 can be let out and the balloon 30 can be contracted.

In the present modification, by expanding the balloon 30, a part of the organ Z can easily be lifted. In addition, by expanding the balloon 30 in the hole that is made when the organ Z is punctured with the sheath 10, bleeding can easily be coagulated.

### [Fourth Embodiment]

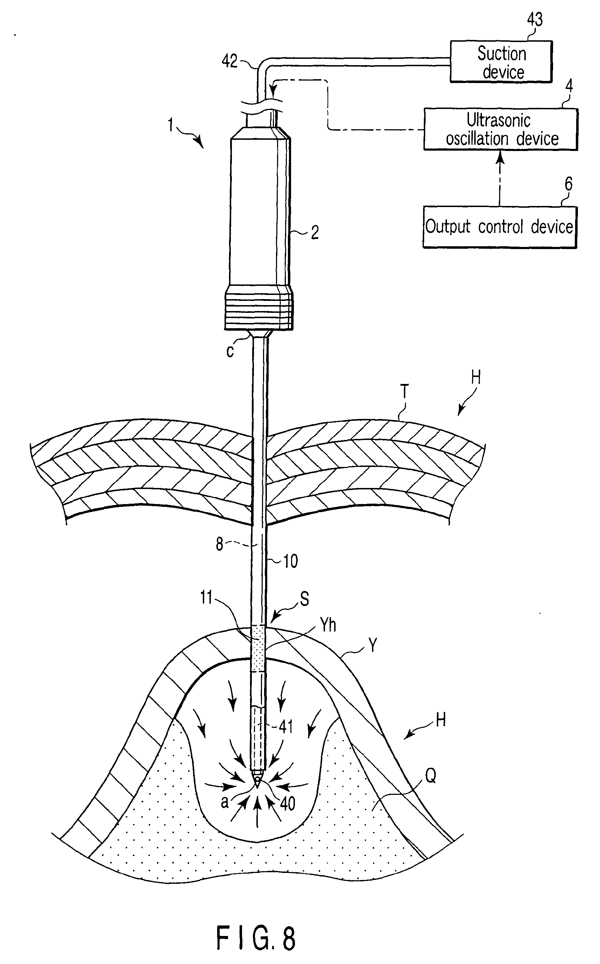

FIG. 8 shows a fourth embodiment of the ultrasonic treatment device 1 of the present invention. The basic structure of the ultrasonic treatment device 1 of this embodiment is the same as that of the first embodiment (see FIG. 1A to FIG. 4B). The parts common to those in FIG. 1 are denoted by like reference numerals, and a description thereof is omitted.

The ultrasonic treatment device 1 of the fourth embodiment is suitable for treating, for example, a diseased ovary. In the case where the ovary is diseased, a cystoma with high viscosity, which is so-called chocolate cystoma, is present within the ovary. The ultrasonic treatment device 1 of this embodiment can be used in order to remove the chocolate cystoma from the ovary.

In the ultrasonic treatment device 1 of this embodiment, a suction hole 40 is formed in the puncture portion a of the probe 8 that projects from the sheath 10. A guide hole 41 is provided within the probe 8. The guide hole 41 is provided along the axis of the probe 8 from the puncture portion a at the distal end of the probe 8 to the insertion guide portion c. The distal end of the guide hole 41 communicates with the suction hole 40.

One end portion of a tube 42 is connected to a rear end of the handpiece 2. This one end portion of the tube 42 communicates with a proximal end portion of the guide hole 41. The other end portion of the tube 42 is connected to a suction device 43, such as a vacuum pump, which is disposed outside the ultrasonic treatment device 1.

Next, the operation of the ultrasonic treatment device 1 according to this embodiment with the above structure is described. Like the first embodiment, when the ultrasonic treatment device 1 of this embodiment is used, the probe 8 and sheath 10 are inserted through the body wall T and the ovary Y, which is an organ, in the same procedure as in the first embodiment (the above-described first insertion step and second insertion step). The length of the probe 8 and sheath 10 according to this embodiment is designed to be sufficiently greater than the thickness of the body wall T and the ovary Y. The puncture portion a at the distal end of the probe 8 and the anti-slip portion 11, which is the lifting operation portion S provided on the sheath 10, reach the inside of the ovary Y.

In this embodiment, a procedure for removing the sheath 10 from the probe 8 is not performed after the completion of the second insertion step. Without drawing the probe 8 out of the sheath 10, the step for lifting a part of the ovary Y is performed.

In the step of lifting the ovary Y, an operation is performed to pull up the sheath 10 and probe 8 in a direction opposite to the direction of puncture into the body. At the time of the operation of pulling up the sheath 10 and probe 8, it is necessary to move the handpiece 2 and sheath 10 as one piece so as to prevent the sheath 10 from being removed from the probe 8.

In the operation of pulling up the sheath 10 and probe 8, when the anti-slip portion 11 moves through the puncture hole Yh in the wall part of the ovary Y, many minute projections 11a of the anti-slip portion 11 stick in the inner peripheral surface of the puncture hole Yh in the wall part of the ovary Y. In other words, the many minute projections 11a of the anti-slip portion 11 are caught in the inner peripheral surface of the puncture hole Yh in the ovary Y, thus preventing a slip.

In this state, the operation for pulling up the sheath 10 and probe 8 is continued. At this time, the sheath 10 moves in a direction opposite to the direction of puncture in the state in which the many projections 11a of the anti-slip portion 11 are caught in the inner peripheral surface of the puncture hole Yh of the ovary Y. Accordingly, the peripheral wall part of the puncture hole Yh of the ovary Y is pulled up together with the sheath 10 toward the body wall T. As a result, the peripheral wall part of the puncture hole Yh of the ovary Y is lifted toward the body wall T. Thus, the distal end portion of the probe 8 is surely immersed in the chocolate cystoma Q in the ovary Y.

Subsequently, a suction step is begun. In this step, the suction device 43 is driven to suck the chocolate cystoma Q through the suction hole 40 in the puncture portion a of the probe 8. Thereby, exact suction is enabled, and the cystoma in the ovary Y is removed in a relatively short time.

The embodiment with the above structure has the following advantageous effects. Like the first embodiment, with the ultrasonic treatment device 1 of this embodiment, the procedure for passing the probe 8 through the body wall T and ovary Y successively is the puncture using ultrasonic oscillation energy. Therefore, the body wall T and ovary Y can smoothly be punctured with the probe 8 using ultrasonic oscillation, while preventing bleeding.

Further, when the puncture procedure with the probe 8 is performed, only the distal-end puncture portion a of the probe 8 is exposed and the other part of the probe 8 is covered with the sheath 10. Thus, ultrasonic oscillation energy is not excessively applied to the living-body wall part.

Furthermore, by pulling up the sheath 10, which is passed through and positioned in the ovary Y, in a direction opposite to the direction of puncture into the body, the wall part of the ovary Y can surely be raised by the anti-slip portion 11. Thereby, the distal end portion of the probe 8 is surely immersed in the chocolate cystoma Q in the ovary Y. Thus, in the subsequent suction step, the chocolate cystoma Q is sucked through the suction hole 40 in the puncture portion a of the probe 8. Therefore, exact suction is enabled, and the cystoma in the ovary Y can be removed in a relatively short time, and the time for the surgical operation can remarkably be reduced.

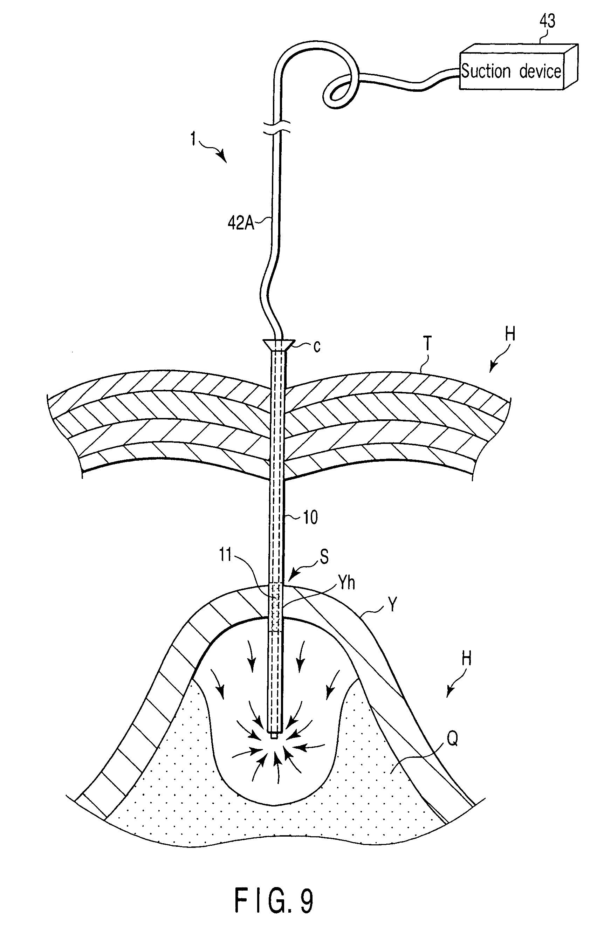

FIG. 9 shows a modification of the ultrasonic treatment device 1 according to the fourth embodiment (see FIG. 8). In this modification, a procedure for removing the sheath 10 from the probe 8 is performed in the ultrasonic treatment device 1 of the fourth embodiment after the completion of the second insertion step of puncturing the body wall T and ovary Y with the probe 8 and sheath 10.

Then, by pulling up the sheath 10, which is passed through and positioned in the ovary Y, in a direction opposite to the direction of puncture into the body, the wall part of the ovary Y can surely be raised by the anti-slip portion 11.

In this state, a suction tube 42A is inserted into the sheath 10 from the funnel-shaped insertion guide portion c. A distal end portion of the suction tube 42A is brought to substantially the same position as the distal end of the sheath 10.

Subsequently, a suction step is begun. In this step, the suction device 43 is driven to suck the chocolate cystoma Q through the suction tube 42A. In this modification, like the fourth embodiment, the chocolate cystoma Q can completely be sucked.

The modification with the above structure has the following advantageous effects. The ultrasonic treatment device 1 of this embodiment, like the ultrasonic treatment device 1 of the fourth embodiment, is applicable to, e.g. the diseased oval Y, and is suitable for suction of so-called chocolate cystoma Q.

In this modification, in particular, after the completion of the second insertion step of puncturing the body wall T and ovary Y with the probe 8 and sheath 10, the sheath 10 is removed from the probe 8. Thus, the inside of the ovary Y is not hurt by the distal end of the probe 8, and a safer operation is realized.

In this modification, there is no need to provide the guide hole 41 that extends from the distal-end puncture portion a of the probe 8 to the insertion guide portion c. Therefore, this modification is advantageous in terms of cost.

Although not shown, if the modification is configured to perform water feeding at the same time as the suction of the chocolate cystoma Q, the suction performance can be enhanced.

Each of the above-described embodiments is configured to lift the part of the organ Z by means of the lifting operation portion S that is disposed at the distal end portion of the sheath 10. The present invention, however, is not limited to this configuration. For example, the lifting operation portion S may be configured to lift a punctured part of the body wall T after the body wall T is punctured with the sheath 10.

Needless to say, the present invention is not limited to the above-described embodiments, and various modifications may be made without departing from the spirit of the invention.

### Industrial Applicability

As has been described above, the present invention is advantageously applicable to a field of ultrasonic treatment devices that are useful in performing treatments, such as coagulation, section and suction, for a diseased part using ultrasonic oscillation, a field of manufacturing and using the ultrasonic treatment devices, and a technical field of treatment methods using the ultrasonic treatment devices.

## Claims

1. An ultrasonic treatment device **characterized by** comprising:
a handpiece (2) accommodating an ultrasonic oscillator (3) that generates ultrasonic oscillation;
a probe (8) including a shaft-shaped oscillation transmission member having a distal end portion and a proximal end portion, the proximal end portion being connected to the ultrasonic oscillator (3), the distal end portion having a puncture portion (a) that transmits the ultrasonic oscillation from the ultrasonic oscillator (3) to a living-body wall part (H) and punctures the living-body wall part (H);
a sheath (10) including a cylindrical member having a distal end portion and a proximal end portion, the cylindrical member being detachably fitted on the probe (8) such that only the distal end portion of the probe (8) is exposed and the other part of the probe (8) is covered, the sheath (10) together with the probe (8) being passed through the living-body wall part (H) by puncture; and
a lifting operation portion (S) provided on the sheath (10), the lifting operation portion (S) being engaged with the living-body wall part (H) at a time of performing a pull-up operation of pulling up the sheath (10) in a direction opposite to a direction of puncture through the living-body wall part (H), thereby performing an operation of lifting the living-body wall part (H).

2. The ultrasonic treatment device according to claim 1, **characterized in that** the probe (8) has at least a length that is needed for penetration through the living-body wall part (H) including a body wall (T) and an internal organ (Z, Y).

3. The ultrasonic treatment device according to claim 1, **characterized in that** the lifting operation portion (S) includes an anti-slip portion (11) with a continuous projection-and-recess shape, which is provided on an outer peripheral surface of the sheath (10).

4. The ultrasonic treatment device according to claim 1, **characterized in that** the lifting operation portion (S) includes:
a bend section (15) that is provided on an outer peripheral surface of the sheath (10), the bend section (15) including a plurality of bend pieces (16) that are juxtaposed in an axial direction of the sheath (10), the bend pieces (16) being rotatably coupled to each other;
an operation wire (17) having a distal end portion and a proximal end portion, the distal end portion of the wire (17) being connected to that one of the bend pieces (16), which is disposed at a foremost position in the axial direction of the sheath (10), the proximal end portion of the wire (17) extending toward the proximal end portion of the sheath (10); and
a bend operation part (19) that is provided on the proximal end portion side of the sheath (10), the bend operation part (19) executing an operation of pulling the wire (17), thereby bending the bend section (15).

5. The ultrasonic treatment device according to claim 1, **characterized in that** the lifting operation section (S) includes a deformable portion (20) in which a plurality of slits (20a) that extend in an axial direction of the sheath (10) are juxtaposed in a circumferential direction of the sheath (10), the deformable portion (20) changing a state of wall portions, which are provided between the slits (20a), between a non-deformed state in which the wall portions are in a straight shape and a projecting state in which the wall portions project outward, thereby varying a maximum outside diameter of the sheath (10).

6. An ultrasonic treatment method **characterized by** comprising:
an insertion step of inserting a probe (8), which is capable of transmitting ultrasonic oscillation to a living-body wall part, and a sheath (10) that covers the probe (8), through the living-body wall part (H); and
a lifting step of lifting the living-body wall part (H) by a lifting operation portion (S) for lifting the living-body wall part (H), the lifting operation portion (S) being provided on the sheath (10), which is inserted into the body through the living-body wall part (H) in the insertion step.

7. The ultrasonic treatment method according to claim 6, **characterized in that** the insertion step includes:
a first insertion step of inserting the probe (8), which is capable of transmitting ultrasonic oscillation to the living-body wall part (H), and the sheath (10) that covers the probe (8), through a body wall part (T) of the living-body wall part (H);
a second insertion step of inserting the probe (8) and the sheath (10), which are inserted into the body through the body wall part (T) in the first insertion step, into an internal organ (Z, Y) that is located inside the body wall part (T); and
a lifting step of lifting the internal organ (Z, Y) by a lifting operation portion (S) for lifting the internal organ (Z, Y), the lifting operation portion (S) being provided on the sheath (10), which is inserted in the second insertion step.
